# EUROPEAN PATENT APPLICATION

(11) **EP 1 779 888 A1**
(43) Date of publication of application: **02.05.2007**
(21) Application number: 06255487.8
(22) Date of filing: 25.10.2006
(51) Int. Cl.: A61M 39/22, A61M 5/168, F16K 41/10, F16K 1/00, F16K 41/12

(54) **Dynamic hermetic barrier for use with implantable infusion pumps**

(30) Priority: 26.10.2005 US 259413
(71) Applicant: Codman & Shurtleff, Inc., Raynham, Massachusetts 02767 (US)
(72) Inventor: Bork, Toralf, 2000 Neuchatel (CH)
(74) Representative: Tunstall, Christopher Stephen

(57) **Abstract**

A valve mechanism (100) for use in an implantable infusion pump includes a fluid compartment and a dry-component compartment. The compartments are sealed so that fluid cannot pass between compartments. A flexible membrane (102) is located between the compartments and allows limited mechanical displacement between the compartments, yet prevents any fluid communication therebetween. The fluid compartment includes a valve that is positioned between the inlet chamber and the outlet chamber. The valve includes a movable trigger member (27) that selectively causes the valve to move between an open position and a closed position. The trigger member is positioned adjacent to the first surface (114) of the membrane. The dry-component compartment includes an actuator (55), which is positioned against the membrane so that generated movement of the actuator may selectively transfer to the trigger member through non-invasive deformation of the flexible membrane. In this arrangement, the valve located within the hermitically-sealed fluid compartment is effectively controlled from the dry component compartment. The flexible membrane includes at least one deformed region (104) that extends beyond the membrane plane, which can be ripple-shaped or bellows-shaped.

## Description

### Background of the Invention

### 1) Field of the Invention:

This invention generally relates to hermetically-sealed devices that have a sealed barrier (or membrane), and more particularly to such hermetically-sealed devices that include mechanical interaction across the barrier.

### 2) Discussion of Related Art:

A variety of mechanical and electro-mechanical devices must operate in environments that require the devices to be completely isolated within a protective barrier. In some situations, the "outside environment" (environment located outside the barrier) is hazardous and includes elements or conditions that will adversely affect the operation of the device or shorten its expected useful operative life. In such hazardous environments, the device must be completely sealed and the protective barrier must be made with the particular hazard in mind. For example, in a chemical-production facility, a temperature sensor may have an operative environment that includes a caustic base chemical. In this harsh environment, the relatively delicate temperature sensor would have to be protected from the strong corrosive chemical in such a way that would not hinder or unpredictably affect its temperature-sensing operation.

In other situations, the outside environment is to be protected from potential danger that stems from within the device itself. For example, if an electrical control device of the type that may create a spark of electricity during its operation is to operate within a combustible environment (e.g., explosive gas), the electrical components of the device will have to be completely isolated (hermetically sealed) from the outside environment.

Also, many medical devices that are to be implanted within a human patient, for example, must also be hermetically sealed, primarily, in this delicate living environment, to prevent infection or contamination caused from within the device.

In all these situations, a protective barrier is created to isolate an outside environment from an inside one. Obviously, different devices and environments require different types and levels of seals. For most non-mechanical interactive devices (that is, devices that do not require cross-barrier mechanical interaction), creating the required hermetic seal is fairly straightforward. The device is typically encased within a plastic or metal housing, which is then sealed using any of a variety of techniques and materials. A commonly used method for sealing electrical circuits that do not require access or air-cooling, is to encase the entire device in a "pot" (or small plastic or metal housing) of epoxy or another appropriate air-tight adhesive or polymer. Other devices may be housed within a plastic or metal housing that is later sealed using rubber sheet, rubber grommets, rubber O-rings, gaskets, adhesives, or metal welding or crimping to ensure that no air can cross the barrier.

These sealing methods and materials work well when no mechanical interaction is required during the life of the device. However, should the device require cross-barrier mechanical interaction, such as linear, angular, or rotational displacement, then the barrier must accommodate the mechanical displacement and the corresponding mechanical stress and fatigue that will likely occur. For example, a good flashlight typically includes a water-tight inside environment that houses the electrical components (battery, bulb, switch, and circuit). A user located in the outside environment must activate the switch to operate the flashlight. To ensure a watertight condition, the inner and outer environments are sealed with a barrier. To allow a user to mechanical displaced the switch across the barrier without creating a leak, a flexible rubber boot is typically positioned over the switch. The flexible boot allows for the switch's mechanical operation, but maintains barrier integrity to the inner environment, as necessary.

Rubber and flexible plastic boots and membranes are often used to accommodate mechanical interaction across a hermetic barrier of many devices. Unfortunately, such rubber and plastic structures, albeit tough and resistant, fail in extreme environmental conditions, including environments having any of a variety of chemicals, radiation, positive and negative pressures, mechanical abrasion, sunlight, and high temperatures (above 300 degrees F) and those below freezing.

One such harsh environment is that of an autoclave, wherein steam is used to sterilize devices intended to be used within or in connection with a human patient. The super-heated water can easily destroy or severely damage such soft flexible non-metallic materials, even before they reach their intended operative environment.

To this end, implantable devices are typically housed within a sturdy metal case and sealed with metal welds to create a very strong and effective barrier that can easily withstand the otherwise harsh conventional sterilization processes. To provide mechanical "communication" with the device across this impervious barrier, a thin membrane of metal is provided at the point of interaction. A common arrangement includes providing a "communication port" (an opening or portal) in the housing and positioning a thin flat flexible metal disc across the port. The metal disc is typically made from titanium and is brazed or soldered (low-heat welding) or high-heat welded to the surrounding metal housing so that the port is effectively sealed and the integrity of the barrier is maintained. The attached disc may be flexed slightly so that a mechanical displacement between the outside environment and a mating component located within the housing may interface, without direct contact (owing to the interposed metal disc).

The flat metal disc has proved quite useful and effective for many devices and situations, but, in some cases may be limited in operative cycles before succumbing to the persistent mechanically-generated stress and fatigue.

It is a first object of the present invention to provide an effective barrier that allows for cross-barrier mechanical interaction, which overcomes the deficiencies of the prior art.

It is another object of the invention to provide a barrier across a communication port that allows for increased mechanical displacement while minimizing mechanical-related stress and fatigue.

### Summary of the Invention

A valve mechanism for use in an implantable infusion pump includes a fluid compartment and a dry-component compartment. The compartments are sealed so that fluid cannot pass between compartments. A flexible membrane is located within a membrane plane, positioned between the compartments and allows limited mechanical displacement between the compartments, yet prevents any fluid communication therebetween. The fluid compartment includes an inlet chamber which is connected to a supply of pressurized liquid medicant by an inlet conduit, an outlet chamber that is connected to an outlet conduit, and a valve that is positioned between the inlet chamber and the outlet chamber. The valve includes a movable trigger member that selectively causes the valve to move between an open position wherein the liquid medicant flows from the inlet chamber to the outlet chamber, and a closed position, wherein medicant flow is prevented. The trigger member is positioned against the membrane. The dry-component compartment includes a displacement actuator, which is positioned against the membrane so that generated movement of the actuator may be selectively transferred to the trigger member through non-invasive deformation of the flexible membrane. In this arrangement, the valve located within the hermitically-sealed fluid compartment is effectively controlled from the dry component compartment. The flexible membrane includes at least one deformed region that extends beyond the membrane plane.
According to other embodiments of the invention, the membrane includes at least one concentric ripple (circular-raised, or circular-lowered ridge), or includes a cylindrically-shaped flexible-bellows structure (either upwardly or downwardly directed within the valve assembly).
According to yet another embodiment, a membrane includes a curved peripheral edge that is sized and shaped to snuggly seat within the peripheral brazing groove located within a membrane support disc. This arrangement helps minimize stress to a braze (or weld) line due to membrane flexure.

### Brief Description of the Drawing Figures

Figure 1 is a perspective view of a valve assembly used in an implantable infusion pump (not shown), showing a flat-disc membrane;
Figure 2 is a sectional, perspective view of the valve assembly of Figure 1, showing details of the valve mechanism and the membrane actuator;
Figure 3 is a perspective upper view of a PRIOR ART flat-disc membrane, shown alone;
Figure 4 is a perspective upper view of a membrane having a ripple-shaped structure, shown alone, according to a first embodiment of the invention;
Figure 5 is a perspective upper view of the ripple-shaped membrane of Figure 4, shown mounted within a membrane support plate, according to the first embodiment of the invention;
Figure 6 is a perspective lower view of the ripple-shaped membrane of Figure 5, shown mounted within the membrane support plate, according to the first embodiment of the invention;
Figure 7 is a cross-sectional view of a valve mechanism of a valve assembly showing the ripple-shaped membrane in position between a "wet-side" piston and a "dry-side" actuator pin, according to the first embodiment of the invention;
Figure 8 is a perspective upper view of a membrane having a bellows structure, shown alone, according to a second embodiment of the invention;
Figure 9 is a side view of the bellows-membrane of Figure 8, according to this second embodiment of the invention;
Figure 10 is a perspective upper view of the bellows-membrane of Figure 9, shown mounted within a membrane support plate, according to this second embodiment of the invention;
Figure 11 is a perspective lower view of the bellows-membrane of Figure 10, shown mounted within the membrane support plate, according to the second embodiment of the invention;
Figure 12 is a cross-sectional, perspective upper view of a valve mechanism of a valve assembly, showing the bellows-membrane of Figure 8 in position between a "wet-side" piston and a "dry-side" actuator pin, according to the second embodiment of the invention;
Figure 13 is a perspective view of a flexible membrane having a deep and varied ripple-shaped structure, shown alone, according to a third embodiment of the invention;
Figure 14 is a side view of the flexible membrane having a deep and varied ripple-shaped structure of Fig. 13, shown alone, according to the third embodiment of the invention;
Figure 15 is a cross-sectional, perspective view of a flexible membrane having a deep and varied ripple-shaped structure of Fig. 13, shown alone, according to the third embodiment of the invention;
Figure 16 is a perspective view of a flexible membrane having a shallow ripple structure, shown alone, according to a fourth embodiment of the invention;
Figure 17 is a cross-sectional, perspective view of a flexible membrane having a shallow ripple structure of Fig. 16, shown alone, according to the fourth embodiment of the invention;
Figure 18 is an upper perspective view of the flexible membrane having a shallow ripple structure of Fig. 18, shown mounted to a portal membrane support ring, according to a fifth embodiment of the invention;
Figure 19 is a lower perspective view of the flexible membrane having a shallow ripple structure of Fig. 18, shown mounted to a portal membrane support ring, according to the fifth embodiment of the invention; and
Figure 20 is a cross-sectional, perspective view of the flexible membrane having a shallow ripple structure of Fig. 19, shown mounted to the portal membrane support ring of Fig. 19, showing details of a brazing channel, according to the fifth embodiment of the invention.

### Detailed Description of the Present Invention

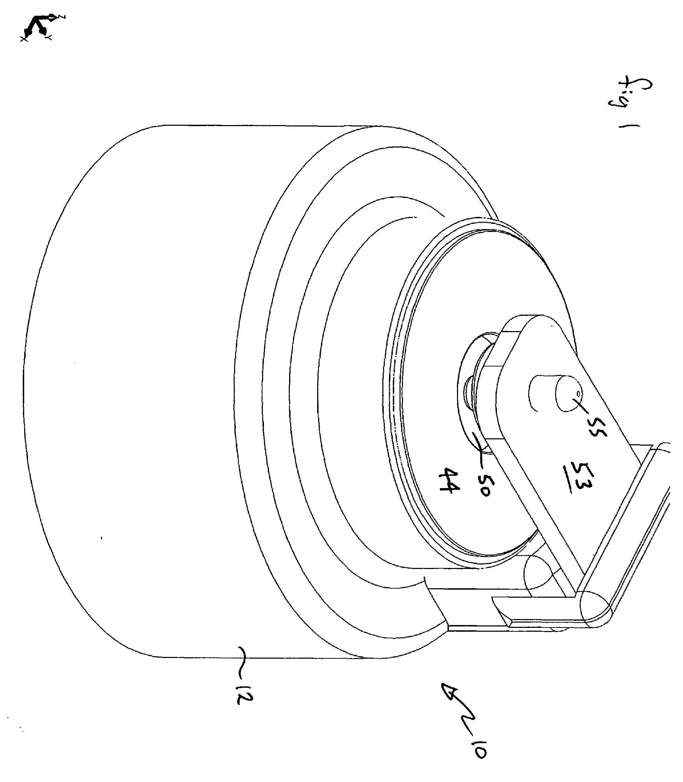

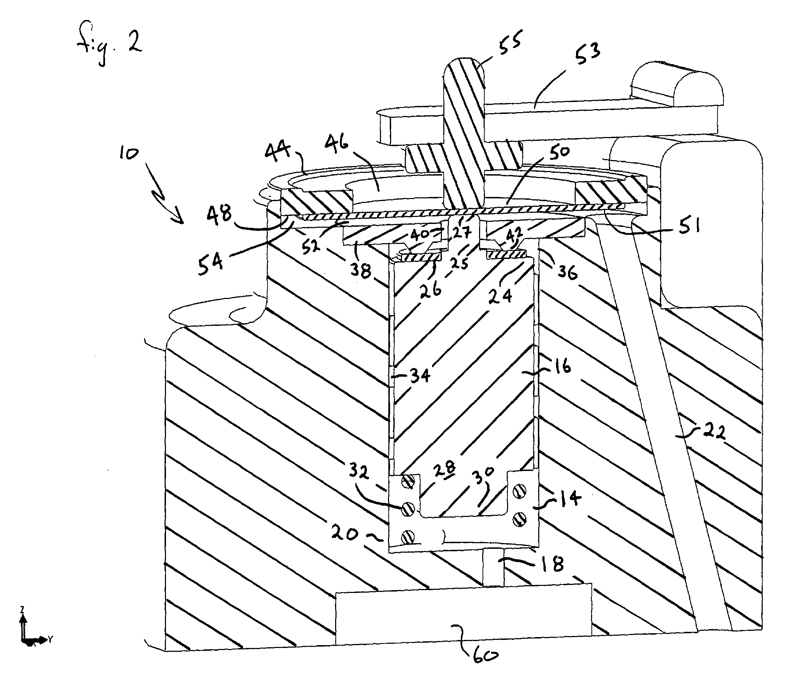

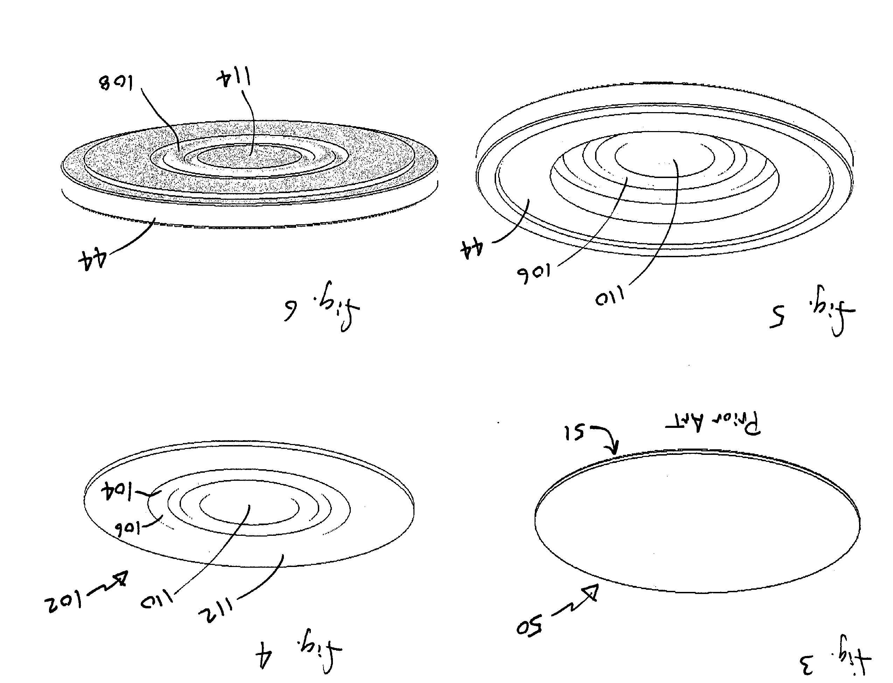

Further details, features and advantages of the invention are shown in the following description of an exemplary embodiment by reference to the drawing.
As discussed above, many devices require isolation from a particular operational environment. The environment is either hazardous to the device, could affect the operation of the device, or, alternatively, the device or supporting materials could cause harm to or affect the operational environment.
In many situations, such isolated devices merely require an appropriate, hermetically-sealed chamber or housing into which the devices are mounted and within which they operate. Some situations, however, require that the housed devices establish a mechanical communication across the "hermetic barrier". The magnitude and frequency of mechanical displacement will, of course, vary depending on the function and type of device, however, some linear or angular displacement across the barrier will be required before, during and/or after the device becomes operational while within the operational environment. In such instance, the chamber or device housing must accommodate mechanical displacement while continuously maintaining its hermetic seal. In fairly harsh environments (such as those of high temperature extremes, or those containing chemical hazards), it is not uncommon to use a barrier that is made from metal, such as steel, brass, copper, or titanium, depending on the device requirements and the operational environment. If a metal housing is used, the portal for mechanical displacement may include a thinned-wall section, which allows sufficient flexibility to accommodate the required mechanical displacement. Alternatively, the portal will be covered (and sealed) with a metal disc or circular membrane that is made from a suitable material, such as titanium. The disc is made from a strong, flexible material and is made thin enough to endure continuous and repeated flexure during the mechanical communication. The discs are typically welded or brazed in place across the communication portal.
The present invention is directed to improved flexible membranes across mechanical access portals of barriers interfacing two isolated environments (hereafter referred to as a "dry side" and a "wet side"). The terms "dry" and "wet" are used to describe two different sides of a barrier and membrane and are not meant to imply environment conditions. Clearly, as is well known, a hermetic barrier can be used to isolate a variety of environments of different conditions and characteristics. Although the present invention can be used in a variety of situations where two environments are isolated and included with a flexible mechanical interface therebetween, the invention is shown and described in connection with a flexible membrane particularly used in the valve-mechanism of implantable infusion pumps of the type that contain a supply of medication and are implanted within a patient.
Referring to Figures 1 and 2, a valve assembly 10 is shown having a body 12 which defines a bore 14 that is sized and shaped to slidably receive a piston 16, as shown in the cross-sectional view of Fig. 2. Body 12 further includes an inlet passage 18 provides fluid communication between a fluid reservoir (not shown) and a lower end 20 of bore 14. Body 12 also includes an outlet passage 22 that carries fluid from the valve assembly 10 (when the valve is open) to a conduit that brings the fluid to a desired and useful site.
In this exemplary valve structure, piston 16 is positioned within bore 14 and includes an upper sealing end 24 that supports a disk-shaped seal 26. Piston 16 further includes a lower end 28, which includes a downwardly-directed boss 30 that is sized and shaped to receive one end of a compression spring 32. Piston 16 further includes a circumferentially located spiral groove 34 (positioned along the sidewall of the piston and extending the length of the piston), which allows fluid communication between the lower end 20 of bore 14 (and inlet passage 22) and upper sealing end 24 of piston 16. Any fluid entering the lower end 20 of bore 14 (under pressure) may freely advance between the piston 16 and the bore 18 through spiral groove 34.
As shown in Fig. 2, compression spring 32 is positioned between lower end 28 of piston 16 and the lower end 20 of bore 14. As further detailed below, spring 32 biases piston 16 (and disk-shaped seal 26) upwardly towards an upper end 36 of bore 14.
Securely attached (i.e., preferably hermetically sealed) to body 12 and positioned over upper end 36 of bore 14 is a contact disc 38 that is preferably made from a rigid material, such as a metal. Contact disc 38 includes a central opening 40 and an integrally formed, downwardly-directed contact ridge 42. Contact ridge 42 is formed concentrically to central opening 40 and is sized and shaped to fit within bore 14, as shown in Fig. 2. Contact disc 38 is positioned so that contact ridge 42 aligns with disk-shaped seal 26 so that as piston 16 is pushed upwardly by spring 32, disk-shaped seal 26 is pressed into a sealing contact with circular contact ridge 42, which closes the valve assembly, as described in greater detail below.
Attached to upper sealing end 24 of piston 16 is an axially-aligned contact pin 25. Contact pin 25 is sized and shaped to loosely fit and slidably move within central opening 40 of contact disc 38 and is long enough so that an upper contact surface 27 extends and remains above contact disc 38. As described in greater detail below, downward displacement of contact pin 25 causes piston 16 to effectively separate disc-shaped seal 26 from sealing contact of contact ridge 42 of contact disc 38, thereby opening the valve.
Securely affixed to body 12 (i.e., preferably hermetically sealed) and positioned over upper end 36 of bore 14 and contact disc 38 is a portal support ring 44, which includes a large central opening 46 and defines a lower surface 48. Attached to the lower surface 48 and covering the large central opening 46 is a thin, flat coin-like, flexible membrane 50, which is, in this instance, considered prior art (and shown in Figure 3). Membrane 50 is positioned above an upper surface 52 of contact disc 38 a predetermined distance so that a collection space 54 is defined there-between.

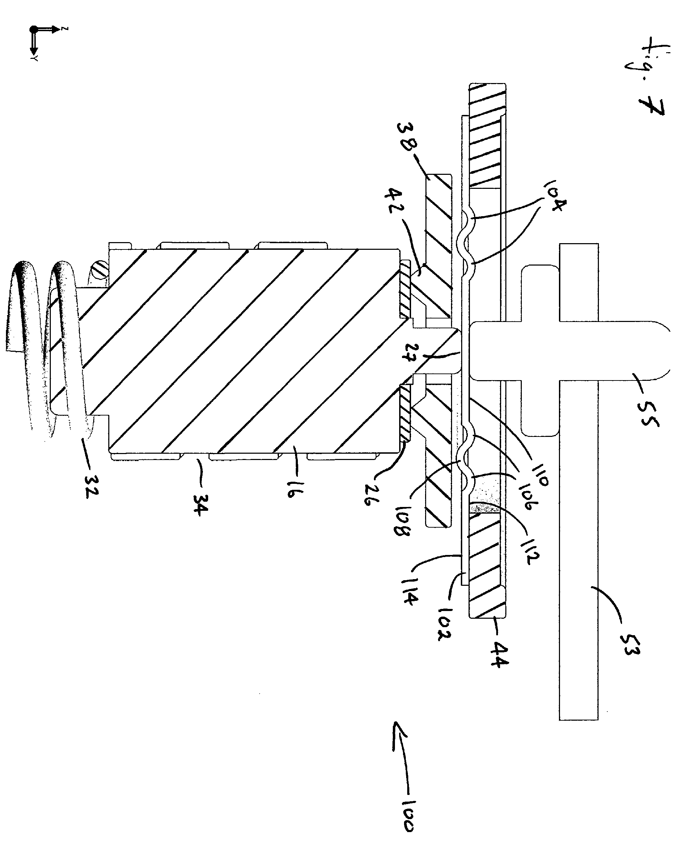

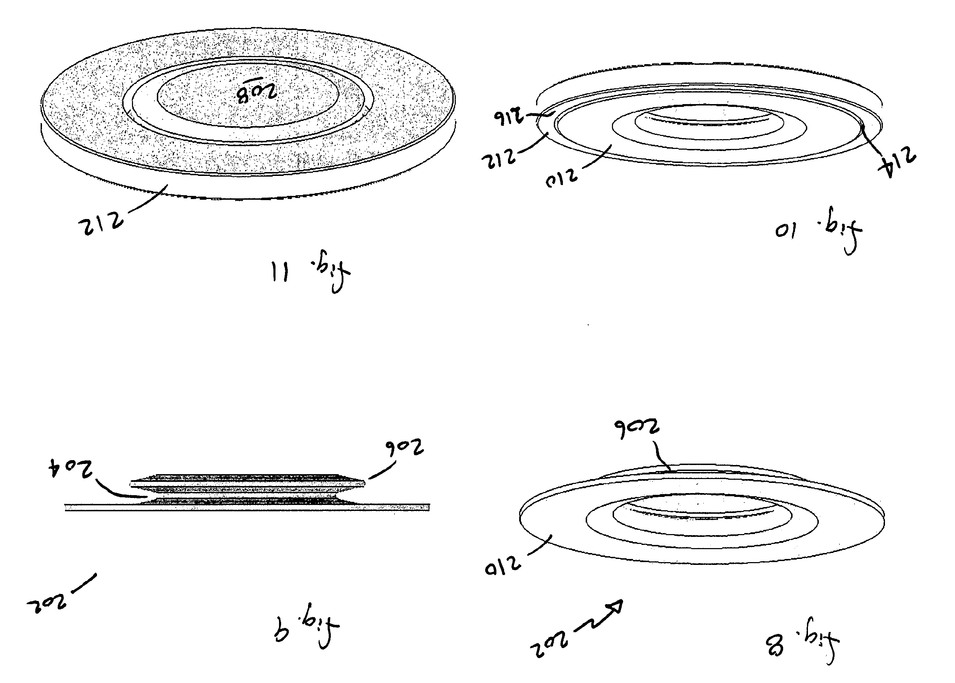

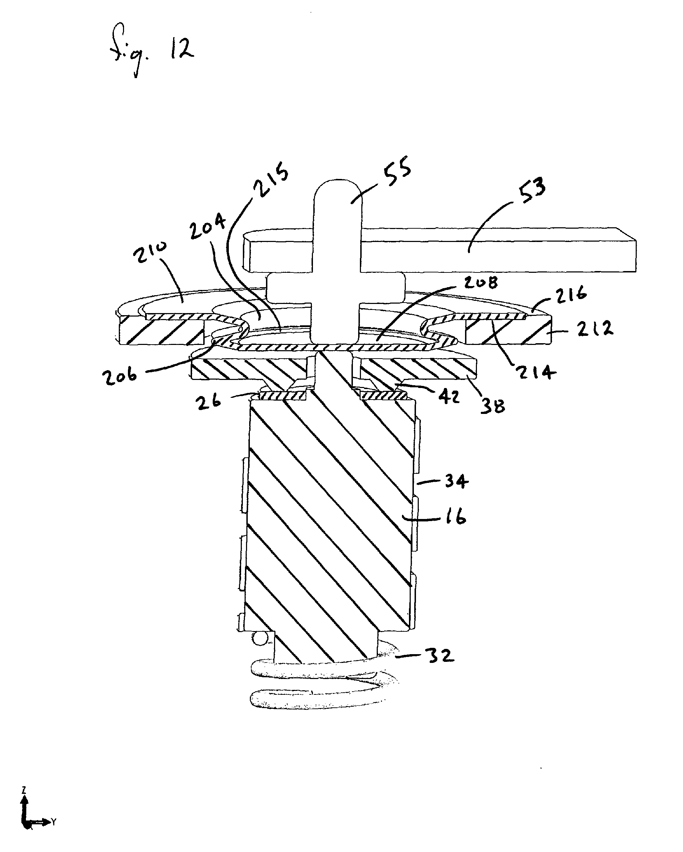

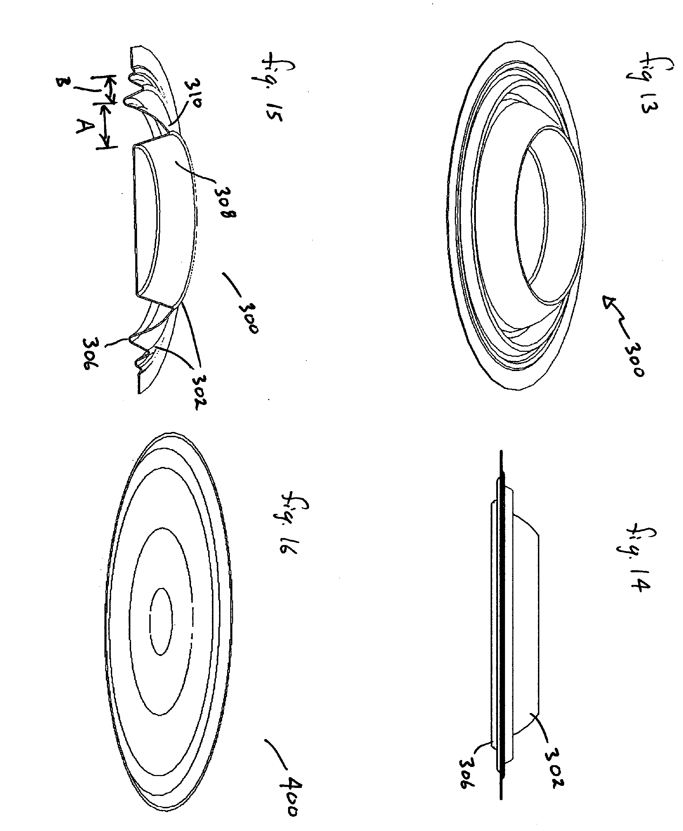

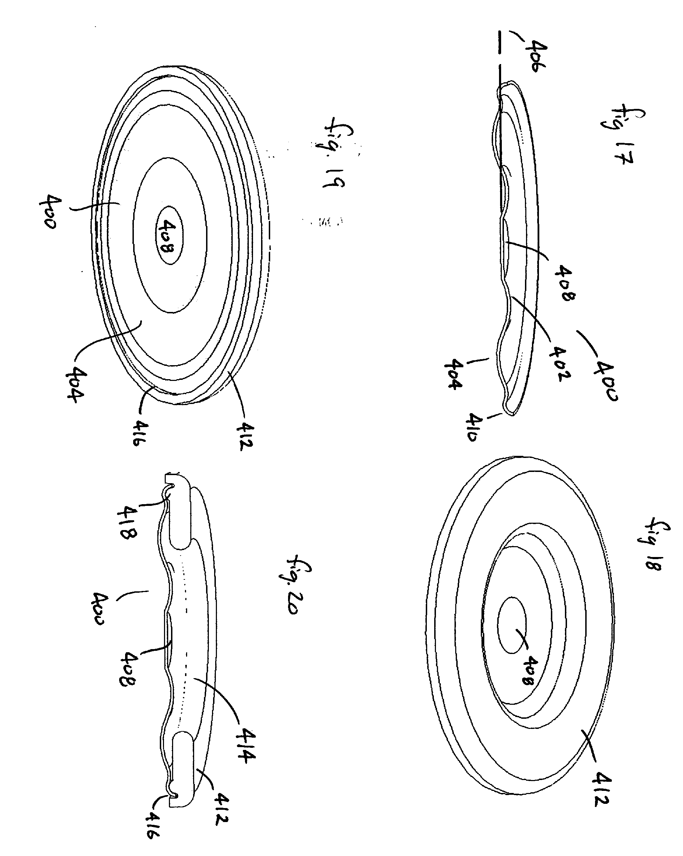

Membrane 50 is usually made from a strong resilient metal, such as titanium and is brazed or welded to the lower surface 48 of portal support ring 44. Similarly, portal support ring 44 is brazed to body 12 so that piston 16, disk-shaped seal 26, spring 32, inlet passage 18, outlet passage 22, and contact disc 38 all define a "wet side" of membrane 50 (lower side) and are all hermetically sealed within the valve body 12 and isolated from everything located above and outside the valve body 12, a space which defines a "dry side" of membrane 50. Membrane 50 is positioned so that upper surface 27 of contact pin 25 abuts against a lower surface 51 of membrane 50. Spring 32 biases contact pin 25 into firm contact with lower surface 51 of membrane 50.
According to this valve application, the valve is opened and closed repeatedly at a predetermined frequency by applying the mechanical displacement generated by a piezo crystal 53 (in response to an applied electrical signal) to move piston 16 up and down. An actuation pin 55 is used to connect the piezo crystal 53 to contact pin 25, indirectly through membrane 50, as described below. Actuation pin 55 is axially aligned with contact pin 25.
In operation of the above described valve assembly 10, fluid (a liquid drug) is supplied to inlet passage 18 under pressure, but regulated by a fluid pressure regulator 60. Fluid enters lower end 20 of bore 14, and whenever piston 16 is forced downwardly within bore 14, against the action of spring 32, fluid from inlet passage 18 moves past piston 16 by way of groove 34 to the top of piston 16. When piston 16 moves downwardly, disc-shaped seal 26 moves away from contact with contact ridge 42, thereby allowing fluid (still under regulated pressure) to pass by contact disc 38 by way of central opening 40, thereafter entering the collection space 54. Any fluid within collection space 54 will be forced into outlet passage 22 and eventually will be directed to a useful site (such as a desired treatment area of a patient's body).
Downward movement of piston 16 is controlled by applying a specific electric signal to the piezo crystal 53, the crystal will deform and will generate a slight downward displacement. This slight downward movement is transferred to the contact pin 25 through the actuation pin 55 and flexible membrane 50. Therefore, the particular electric signal applied to the piezo crystal 53 will indirectly control the opening of the valve assembly 10 and therefore the amount and effective rate of fluid passing from inlet passage 18 to outlet passage 22.
Referring to Figures 4, 5, 6, and 7, a valve assembly 100 (for clarity, shown without a body structure 12) is shown including all the same parts as the valve assembly 10, shown in Figures 1, and 2 and described above, except that the flat, coin-like membrane 50 has been replaced with a membrane 102 which includes at least one integrally formed concentric ripple 104, according to a first embodiment of the invention. Assuming a metal membrane, each ripple 104 is preferably stamped using an appropriate die. The stamping die (not shown) forms at least an upper portion 106 or a lower portion 108, but preferably a series of ripples 104 into membrane 102, as shown in Figures 4 through 7. Concentric ripples 104 define a central contact circle 110 against which actuator pin 55 and contact pin 25 will abut, on upper surface 112 and lower surface 114, respectively, of membrane 104.
In operation of valve assembly 100, as actuation pin 55 is linearly displaced downward against piston 16, by piezo crystal 53, membrane 102 will allow for greater displacement of piston 16 and concentric ripples 104 will help retain such displacement to within contact circle 110. As actuator pin 55 moves downwardly against contact circle 110 of membrane 102, concentric ripples 104 will effectively absorb such axial movement and will thereby attenuate the movement that reaches the peripheral brazed or welded edge 114 of membrane 102. Ripples 104 will therefore increase the effective life of the membrane by preventing damage to the relatively delicate brazed edge 114 between membrane 102 and the portal support ring 44.
Of course the particular dimensions of the rippled membrane 102, including the number and amplitude of the individual ripples 104 and their respective diameters, as well as the thickness and type of material used will vary depending on the particular application intended.
Referring now to Figures 8, 9, 10, 11, and 12, a valve assembly 200, (again, for clarity, shown without a body structure 12) is shown including all the same parts as the valve assembly 10, shown in Figures 1, and 2 and described above, except that the flat, coin-like membrane 50 has now been replaced with a membrane 202 which includes a bellows structure, according to a second embodiment of the invention. As shown in Figures 9 and 12, a bellows membrane 202 includes at least one inward concentric bend 204 and at least one outward concentric bend 206, and further defines a contact circle 208 and a mounting flange 210. Mounting flange 210 is brazed (or welded) to a portal support ring 212 which preferably includes a circular recess 214 on an upper surface 216, as shown in Figures 10 and 12. To help maintain a relatively low-profile valve assembly 200, bellows membrane 202 is preferably mounted to upper surface 214 and portal support ring 212 preferably includes a central opening 215 that is sized and shaped to receive and allow free movement of bellows membrane 202.
In operation of valve assembly 200, as actuation pin 55 is linearly displaced downward against piston 16, by piezo crystal 53, bellows membrane 202 will allow for greater displacement of piston 16 and bellows inward and outward bends 204 and 206, will help retain such displacement to within contact circle 208. As actuator pin 55 moves downwardly against contact circle 208 of membrane 202, the bellows structure will effectively absorb such axial movement and will thereby attenuate the magnitude of movement that reaches the mounting flange (and therefore the brazed or welded edge). This bellows structure of membrane 202 will therefore increase the effective life of the membrane by preventing damage to the relatively delicate brazed (or welded) edge between membrane 202 and the portal support ring 44.
Of course the particular dimensions of the bellows structure including the number of inward bends 204 and outward bends 206 and their respective inside diameters, as well as the thickness and type of material used will vary depending on the particular application intended. It should be noted that although the membranes of the present invention have been described in connection with an on-board control valve assembly as part of an implantable drug-infusion pump, the membranes of the present invention may be effectively applied to any hermetically sealed barrier for the purpose of allowing controlled mechanical interaction across the barrier without disrupting the hermetic qualities of the barrier.
According to a fourth embodiment of the invention, referring to Figures 13, 14, and 15, a varied-rippled membrane 300 is shown having upper ripples 302 that vary in height above a membrane plane 304, and lower ripples 306 that vary in depth below the membrane plane 304. Also, as shown in the figures, the walls of any ripple (302, 306) may be straight (as shown by wall 308 of Figure 15), or curved (as shown by wall 310, of Figure 15). Also, according to this fourth embodiment of the invention, the radial distance between each ripple (302, 306) may vary (as illustrated by letters "A" and "B" in Figure 15).

According to a fourth embodiment of the invention, referring to Figures 16, 17, 18, 19 and 20, a rippled membrane 400 is shown having an upper shallow ripple 402 and a lower shallow ripple 404, with respect to a membrane plane 406, and a center contact circle 408. According to this fourth embodiment of the invention, membrane 400 further includes a curved peripheral edge 410 (may be curved up or down, but is shown curved up to explain the invention).
A portal membrane mounting ring 412 is shown having a central opening 414, a peripheral brazing groove 416 and a rounded support surface 418. According to the invention, membrane 400 is sized and shaped to snuggly fit into portal membrane mounting ring 412 so that upwardly curved peripheral edge 410 is positioned within brazing groove 416 and against rounded support surface 418. The purpose of this curved peripheral edge 410 and the brazing groove 416 is to discourage stress on the brazing weld caused by repeated flexing movement of the membrane 400. The curved peripheral edge 410 of this fourth embodiment can be applied to any shaped membrane described in this application, as well as the flat, coin-like membrane of the prior art.

## Claims

1. A valve mechanism for use in an implantable infusion pump, comprising:
a fluid compartment and a dry-component compartment, said compartments being sealed so that fluid from said fluid compartment is blocked from entering said dry-component compartment;
a flexible membrane, located within a membrane plane and being positioned between said compartments, said flexible membrane allowing limited mechanical displacement between said compartments, yet preventing any fluid communication therebetween, said membrane having a first surface located within said fluid compartment and an opposing second surface located within said dry-component compartment;
said fluid compartment including:
an inlet chamber connected to a supply of pressurized liquid medicant by an inlet conduit;
an outlet chamber connected to an outlet conduit;
a valve positioned between said inlet chamber and said outlet chamber, said valve including a movable trigger member that selectively causes said valve to move between an open position wherein said liquid medicant may flow from said inlet chamber to said outlet chamber, and a closed position, wherein medicant flow is prevented, said trigger member being positioned adjacent to said first surface of said membrane;
said dry-component compartment including an actuator, said actuator being positioned adjacent to said second surface of said membrane so that generated movement of said actuator may be selectively transferred to said trigger member through non-invasive deformation of said flexible membrane so that said valve located within said fluid compartment may be effectively controlled from said dry component compartment; and
said flexible membrane including at least one deformed region that extends beyond said membrane plane.

2. The valve mechanism according to claim 1, wherein said deformed region of said flexible membrane includes a circular ripple-shaped ridge that physically extends into said dry-component compartment from said membrane plane.

3. The valve mechanism according to claim 1, wherein said deformed region of said flexible membrane includes a circular ripple-shaped ridge that physically extends into said fluid compartment from said membrane plane.

4. The valve mechanism according to claim 1, wherein said deformed region of said flexible membrane includes a first circular ripple-shaped ridge that physically extends into said dry-component compartment from said membrane plane and a second circular ripple-shaped ridge that physically extends into said fluid compartment from said membrane plane.

5. The valve mechanism according to claim 4, wherein said first circular ripple-shaped ridge has a first radius from the center of said membrane and second circular ripple-shaped ridge has a second radius from said membrane center, said first radius is less than said second radius.

6. The valve mechanism according to claim 5, wherein said first radius is greater than said second radius.

7. The valve mechanism according to claim 1, wherein said deformed region of said flexible membrane includes a first and third circular ripple-shaped ridge that physically extends into said dry-component compartment from said membrane plane and a second circular ripple"-shaped ridge that physically extends into said fluid compartment from said membrane plane.

8. The valve mechanism according to claim 7, wherein said second circular ripple-shaped ridge is radially positioned between said first and third ridges, with respect to the center of said membrane.

9. A valve mechanism for use in an implantable infusion pump, comprising:
a fluid compartment and a dry-component compartment, said compartments being sealed so that fluid from said fluid compartment is blocked from entering said dry-component compartment;
a membrane-support ring positioned between said compartments, said support ring including a dry-side which lies within said dry-component compartment, and an opposing fluid-side which lies within said fluid compartment, and a central opening;
a flexible membrane, mounted to said membrane-support ring, across said central opening and located within a membrane plane, said flexible membrane allowing limited mechanical displacement between said compartments, yet preventing any fluid communication therebetween, said membrane having a first surface located within said fluid compartment and an opposing second surface located within said dry-component compartment;
said fluid compartment including:
an inlet chamber connected to a supply of pressurized liquid medicant by an inlet conduit;
an outlet chamber connected to an outlet conduit;
a valve positioned between said inlet chamber and said outlet chamber, said valve including a movable trigger member that selectively causes said valve to move between an open position wherein said liquid medicant may flow from said inlet chamber to said outlet chamber, and a closed position, wherein medicant flow is prevented, said trigger member being positioned adjacent to said first surface of said membrane;
said dry-component compartment including an actuator, said actuator being positioned adjacent to said second surface of said membrane so that generated movement of said actuator may be selectively transferred to said trigger member through non-invasive deformation of said flexible membrane so that said valve located within said fluid compartment may be effectively controlled from said dry component compartment; and
said flexible membrane including at least one deformed region that extends beyond said membrane plane.

10. The valve mechanism according to claim 9, wherein said deformed region of said membrane is a cylindrical bellows structure that includes at least one inward concentric bend and one outward concentric bend.

11. The valve mechanism according to claim 10, wherein said bellows structure includes a peripheral mounting flange that remains within said membrane plane, said peripheral mounting flange being affixed to said fluid surface of said membrane-support disc.

12. The valve mechanism according to claim 10, wherein said bellows structure includes a peripheral mounting flange that remains within said membrane plane, said peripheral mounting flange being affixed to said dry surface of said membrane-support disc and said bellows structure being sized and shaped to slidingly displace within said central opening of said membrane-support ring.

13. The valve mechanism according to claim 10, wherein said bellows structure includes a peripheral mounting flange that remains within said membrane plane, said peripheral mounting flange being affixed to said fluid surface of said membrane-support disc and said bellows structure being sized and shaped to slidingly displace within said central opening of said membrane-support ring.

14. The valve mechanism according to claim 10, wherein said bellows structure includes a peripheral mounting flange that remains within said membrane plane, said peripheral mounting flange being affixed to said dry surface of said membrane-support disc.

15. The valve mechanism according to claim 10, wherein said bellows structure includes a peripheral mounting flange that remains within said membrane plane, said peripheral mounting flange being affixed to said fluid surface of said membrane-support disc.

16. The valve mechanism according to claim 9, wherein said membrane support disc further includes a peripheral brazing groove and said membrane includes a curved edge that is sized and shaped to snuggly fit within said brazing groove.

17. The valve mechanism according to claim 16, wherein said membrane support disc further includes a concentric, rounded support ridge located adjacent to said peripheral brazing groove, said support ridge being sized and shaped to snuggly and supportingly receive said curved edge of said membrane.

18. A membrane for use within a valve assembly of an implantable infusion pump, of the type that controls the flow of medicant from a medicant reservoir to a desired site within a patient by opening and closing a valve in response to applied mechanical displacement generated by an actuator located outside said valve assembly and being applied to said valve through said membrane, said membrane defining a membrane plane and comprising:
a dry surface which lies within a dry-compartment of said valve assembly;
a fluid surface which lies within a fluid-compartment of said valve assembly;
a pre-deformed shape extending beyond said membrane plane, said pre-deformed shape providing extended flexibility to said membrane during transfer of said mechanical displacement from said actuator to said valve.

19. The membrane of claim 18, wherein said pre-deformed shape includes at least one concentric ridge.

20. The membrane of claim 18, wherein said pre-deformed shape includes a cylindrical bellows structure.
